Europäisches Patentamt

European Patent Office

Office européen des brevets

(19)

(11) Publication number: **0 103 570**
**B1**

(12) **EUROPEAN PATENT SPECIFICATION**

(45) Date of publication of patent specification: **27.08.86**

(21) Application number: **82900838.2**

(22) Date of filing: **15.03.82**

(86) International application number:
**PCT/SE82/00069**

(87) International publication number:
**WO 83/03307 29.09.83 Gazette 83/23**

(51) Int. Cl.⁴: **G 01 N 33/531,** G 01 N 33/74
// A61K39/395

(54) **METHOD FOR PRODUCTION OF ANTISERUM AGAINST GRANULA FRACTION FROM ENDOCRINOLOGICALLY ACTIVE CELLS.**

(43) Date of publication of application:
**28.03.84 Bulletin 84/13**

(45) Publication of the grant of the patent:
**27.08.86 Bulletin 86/35**

(84) Designated Contracting States:
**CH DE FR GB LI**

(56) References cited:
**SE-A- 423 456**

**Histochemistry Vol 52, pp 217-22, published 1977 (ALUMETS J et al) "Substance P and 5-HT in Granules Isolated from an Intestinal Argentaffin Carcinoid"**
**Chemical Abstracts, Vol 69 (1968), abstract No 58047m, Histochemie 1968, 13, 323-30**

(73) Proprietor: **MILAB MALMÖ IMMUNLABORATORIUM AB Box 20047 S-200 74 Malmö (SE)**

(72) Inventor: **HAKANSSON, Rolf Tunnbindaregatan 8 S-222 36 Malmö (SE)**
Inventor: **THORELL, Jan Beleshögsvägen 1 S-216 18 Malmö (SE)**

(74) Representative: **Ström, Tore et al Ström & Gulliksson AB Studentgatan 1 P.O. Box 4188 S-203 13 Malmö (SE)**

Courier Press, Leamington Spa, England.

## Description

1. The invention relates to a method for manufacturing a substance for identifying predetermined endocrinally active cells.

Cells producing peptide hormones are characterised by the ability thereof to synthetise and secrete different types of biologically active polypeptides, i.e. hormones. The hormones are frequently produced within specific parts of the cells, the so-called ribosomes, in the form of large precursor molecules, termed prohormones, which are stored and are transformed to the final form of the hormones in other parts of the cells, usually in specific cavities or vesicles which are separated from surrounding cytoplasm by a diaphragm. These cavities or vesicles are frequently termed granules after the appearance thereof when examined microscopically. During the transformation of the precursor molecules or prohormones to the final hormone a number of splitting products appear. Known prohormones which are stored in cell granules and are converted to hormones therein, include proinsulin, ACTH lipotropic precursor and so-called big gastrin. In cell granules there are in addition to structure proteins also other active proteins, e.g. enzymes, which participate in the conversion of prohormones to hormones.

For the identification of endocrinally active cells in histological sections a long-established technique exists utilising the fact that antibodies (immune globulines) against the hormones are bound to these cells. Then, the antibodies can be demonstrated by coupling to them either a fluorescent substance, e.g. fluorescein-isothiocyanate, or an enzyme which can be visualised by means of suitable substances.

However, for some endocrinally active cells no suitable identification methods exist since the technique mentioned above cannot be applied due to the fact that it has not been possible so far to produce antibodies against the hormones which are stored in the related cells.

The purpose of the invention is to provide a substance which can be used for identifying said latter type of endocrinally active cells, and for this purpose the invention provides a method of manufacturing an antiserum anti-peptide- or anti-protein-containing insoluble granule fraction from endocrinally active cells for the identification of such cells by applying the antiserum to microscopic sections, which is characterised in that tissue with said endocrinally active cells is disintegrated, that an insoluble granule fraction including a peptide or protein component emanating from the cytoplasm, and having a particle size of 800 nm is separated from the disintegrated product in a manner known per se, that the insoluble granule fraction is suspended in a water solution, and that the antiserum is manufactured by using the water suspension thus obtained.

According to Histochemistry 52 217—222 (1977) it is known to separate an insoluble granule fraction, by homogenising the disintegrated product, cleaning the homogenised product from cells, cell nuclei, and other major fragments by centrifugation, and filtrating the cleaned product.

Histochemie 13, 323—330 (1968) describes a method of producing an antiserum from an ox chromaffin granule soluble protein fraction for use in an indirect immunohistochemical technique.

Particularly, it is intended to isolate by the method of the invention a peptide or protein component from such endocrinally active cells which could not be identified earlier by applying the common technique, and to produce antisera against said component, which can then be applied to microscopic histological sections in order to prove the presence of such cells in the section.

The invention is illustrated below by the description of the application of the method to specific endocrinally active cells in the gastro-intestinal canal, appertaining to the so-called enterochromaffin cell system.

Since the peptide hormone producing cells in the gastro-intestinal canal are not concentrated but are diffusely scattered great difficulties are involved in isolating individual peptides from such cells. Therefore, in the illustrative application the possibility of isolating the related cell components from a tumor, initiated from cells of the tumor group identified as substance P producing carcinoid, has been chosen.

The tumor cells were disintegrated by homogenisation in a 0.3 M sucrose solution, and the homogeneous product obtained was cleaned from cells, cell nuclei and other major fragments by centrifugation at 1000×g for twenty minutes at 0°C. The supernatant was passed through an array of Millipore® filters having decreasing pore size: 8000, 5000, 3000, 1200, and 800 nm. The filtrate, an insoluble fraction with a particle size less than 800 nm was then centrifugated at 40000×g for thirty minutes. The sediment or pellet thus obtained was dispersed in distilled water, dialysed against water for twentyfour hours and lyophilised.

The lyophilised material was then suspended in a water solution and homogenised, so as to provide a suspension consisting of soluble as well as insoluble components of the walls and contents of the granules. A number of rabbits were immunised by intracutaneous injection of the suspension together with Freund's adjuvant. After six weeks antibodies against a component in the suspension could be demonstrated by the fact that tumour cells from substance P producing carcinoids reacted at conventional immunohistochemical staining.

At present the antigen which elicits the antibodies in the obtained antiserum has not been definitely identified.

When applying the antiserum to histological sections from a normal gastro-intestinal canal it has been found that the antibodies can be bound to the endocrinally active cells from which substance P producing carcinoids are considered to emanate. A series of carcinoid tumours has been examined with PAP-labelled antiserum manufactured as described above, and it has been found that such tumours react positively with the antiserum. This does not react, however, with cells from tumours of other origin.

### Claims

1. Method of manufacturing an antiserum anti-peptide- or anti-protein-containing insoluble granule fraction from endocrinally active cells for the identification of such cells by applying the antiserum to microscopic sections, characterised in that tissue with said endocrinally active cells is disintegrated, that an insoluble granule fraction including a peptide or protein component emanating from the cytoplasm, and having a particle size of 8800 nm, is separated from the disintegrated product in a manner known per se, that the insoluble granule fraction is suspended in a water solution, and that the antiserum is manufactured by using the water suspension thus obtained.

2. Method as claimed in claim 1, characterised in that the tissue which is disintegrated consists of tumours from said cells.

3. Method as claimed in claim 1 or 2, characterised in that the insoluble granule fraction is centrifuged and that the sediment obtained is dispersed in water and is lyophilised before being suspended in the water solution.

4. Method as claimed in any of claims 1—3, characterised in that the granule fraction is manufactured from tumours of the type substance P producing carcinoid.

### Patentansprüche

1. Verfahren zur Herstellung eines Antiserums gegen eine Antipeptid- oder Antiprotein-enthaltende, unlösliche Fraktion von Körnchen aus endokrinologisch aktiven Zellen zur Identifizierung solcher Zellen durch Auftrag des Antiserums auf mikroskopische Schnitte, dadurch gekennzeichnet, daß das Gewebe mit den endokrinologisch aktiven Zellen zersetzt wird, daß eine unlösliche Fraktion von Körnchen einschließlich einer Peptid- oder Protein-Komponente, die von dem Zytoplasma eminiert, und eine Partikelgröße von 800 nm hat, aus dem zerzetzten Produkt in einer an sich bekannten Weise abgetrennt wird, daß die unlösliche Fraktion von Körnchen in einer Wasserlösung suspendiert wird und daß das Antiserum unter Verwendung der so erhaltenen Wassersuspension hergestellt wird.

2. Verfahren nach Anspruch 1, dadurch gekennzeichnet, daß das Gewebe, das zersetzt wird aus Tumoren aus diesen Zellen besteht.

3. Verfahren nach Anspruch 1 oder 2, dadurch gekennzeichnet, daß die unlösliche Fraktion von Körnchen zentrifugiert wird und daß das erhaltene Sediment in Wasser dispergiert und lyophilisiert wird, bevor es in der Wasserlösung suspendiert wird.

4. Verfahren nach einem der Ansprüche 1 bis 3, dadurch gekennzeichnet, daß die Fraktion von Körnchen aus Tumoren vom Typ eines Substanz P produzierenden Karzinoids hergestellt wird.

### Revendications

1. Procédé de préparation d'une fraction de granulations insoluble contenant un antisérum anti-peptide ou anti-protéine provenant de cellules à activité endocrinienne pour l'identification de ces cellules en appliquant l'antisérum à des coupes microscopiques, caractérisé en ce que ce tissue avec ces cellules à activité endocrinienne est désintégré, en ce qu'une fraction de granulations insoluble comprenant un constituant peptidique ou protéinique émanant du cytoplasme, et ayant une taille de particules de 800 nm est séparée du produit désintégré d'une manière connue en soi, en ce que cette fraction de granulations insoluble est mise en suspension dans une solution aqueuse, et en ce que l'antisérum est préparé en utilisant la suspension aqueuse ainsi obtenue.

2. Procédé selon la revendication 1, caractérisé en ce que le tissu qui est désintégré se compose de tumeurs provenant de ces cellules.

3. Procédé selon les revendications 1 ou 2, caractérisé en ce que la fraction de granulations insolubles est centrifugée et en ce que le sédiment obtenu est dispersé dans de l'eau et est lyophilisé avant d'être mis en suspension dans la solution aqueuse.

4. Procédé selon l'une quelconque des revendications 1 à 3, caractérisé en ce que la fraction de granulations est préparée à partir de tumeurs du type carcinoïde produisant la substance P.